Europäisches Patentamt

European Patent Office

Office européen des brevets

(19)

(11) **EP 1 039 916 B1**

(12) **FASCICULE DE BREVET EUROPEEN**

(45) Date de publication et mention
de la délivrance du brevet:
**10.12.2003 Bulletin 2003/50**

(21) Numéro de dépôt: **98962487.9**

(22) Date de dépôt: **17.12.1998**

(51) Int Cl.⁷: **A61K 31/715**, A61P 1/02

(86) Numéro de dépôt international:
**PCT/FR98/02758**

(87) Numéro de publication internationale:
**WO 99/032099 (01.07.1999 Gazette 1999/26)**

(54) **UTILISATION D'AU MOINS UN FUCANE POUR L'OBTENTION D'UN MEDICAMENT DESTINE AU TRAITEMENT DES PATHOLOGIES PARODONTALES**

VERWENDUNG VON WENIGSTEN EINEM FUKAN ZUR HERSTELLUNG EINES ARZNEIMITTELS FÜR DIE BEHANDLUNG PARIODONTALER ERKRANKUNGEN

USE OF AT LEAST ONE FUCANE FOR THE MANUFACTURE OF A MEDICAMENT FOR THE TREATMENT OF PERIODONTAL PATHOLOGIES

(84) Etats contractants désignés:
**AT BE CH CY DE DK ES FI FR GB GR IE IT LI LU MC NL PT SE**

(30) Priorité: **18.12.1997 FR 9716080**

(43) Date de publication de la demande:
**04.10.2000 Bulletin 2000/40**

(73) Titulaire: **INSTITUT FRANCAIS DE RECHERCHE POUR L'EXPLOITATION DE LA MER (IFREMER) 92138 Issy-les-Moulineaux Cedex (FR)**

(72) Inventeurs:
• **SENNI, Karim**
 **F-93600 Aulnay sous Bois (FR)**
• **PELLAT, Bernard**
 **F-92120 Montrouge (FR)**
• **GOGLY, Bruno**
 **F-77840 Crouy/Ourcq (FR)**
• **BLONDIN, Catherine**
 **F-75012 Paris (FR)**
• **LETOURNEUR, Didier**
 **F-92350 Le Plessis Robinson (FR)**
• **JOZEFONVICZ, Jacqueline**
 **F-60260 Lamorlaye (FR)**

• **SINQUIN, Corinne**
 **F-44300 Nantes (FR)**
• **COLLIEC-JOUAULT, Sylvia**
 **F-44300 Nantes (FR)**
• **DURAND, Patrick**
 **F-44400 Reze-les-Nantes (FR)**

(74) Mandataire: **Vialle-Presles, Marie José et al Cabinet ORES, 36,rue de St Pétersbourg 75008 Paris (FR)**

(56) Documents cités:
 **EP-A- 0 676 207      EP-A- 0 730 867**
 **WO-A-97/08206**

• **M. ELLOUALI ET AL.: "Antiproliferative effect and interaction of fucans with cells." COLLOIDS AND SURFACES, vol. 2, no. 1-3, 1994, pages 305-314, XP002076126**
• **D. LOGEART ET AL: "Collagen synthesis by vascular smooth muscle cells in the presence of antipoliferative polysaccharides." J. BIOMED. MATERIALS RES., vol. 30, no. 4, 1996, pages 501-508, XP002076127**

**Description**

L'invention est relative à de nouvelles utilisations des fucanes dans le cadre de la réparation des lésions du tissu conjonctif, et en particulier pour la régulation des fonctions fibroblastiques.

Les fibroblastes jouent un rôle essentiel dans l'équilibre et la réparation des tissus conjonctifs. Ils sont en particulier responsables du renouvellement de la matrice extra-cellulaire, et leurs fonctions sont modulées en retour par les substances présentes dans cette matrice.

En particulier, dans le processus de cicatrisation et de remodelage tissulaire intervenant après une blessure, le tissu conjonctif est le cadre d'échanges constants entre toutes les cellules intervenant dans ce processus. Ces échanges s'effectuent en particulier par l'intermédiaire de cytokines ou de médiateurs solubles transmis par la matrice extracellulaire.

Par exemple, dans les tissus conjonctifs de recouvrement tels que les tissus gingivaux et cutanés, le processus de cicatrisation débute, après la formation d'une matrice provisoire (thrombus rouge), par le recrutement de cellules inflammatoires (leucocytes, macrophages et polynucléaires), qui initient une phase de destruction du tissu lésé.

Ces cellules inflammatoires participent à la destruction :

- en sécrétant des protéases matricielles telles que la collagénase (MMP8), l'élastase leucocytaire, ou la cathepsine G),
- en libérant des cytokines, et notamment l'interleukine 1(IL1), qui stimulent la prolifération et la migration des fibroblastes et des cellules épithéliales, et l'expression par ces cellules de certaines métalloprotéases, telles que la collagénase interstitielle (MMP1), ou la gélatinase B (MMP9).

Cette phase de destruction qui débute très tôt après la blessure, se termine lorsque l'épithélium et sa membrane basale se sont reconstitués.

Elle est prolongée par des phases de réparation et de résolution où les fibroblastes reconstruisent et réorganisent la trame collagénique ; on observe en particulier l'expression par les fibroblastes de la gélatinase A (MMP2), métalloprotéase matricielle participant activement à tous les phénomènes de remodelage tissulaire.

Certaines pathologies s'accompagnent d'un état inflammatoire chronique du tissu conjonctif, où l'équilibre entre les phases de destruction, de réparation, et de résolution est rompu, ce qui conduit à une reconstruction défectueuse du tissu lésé.

Ce phénomène est en particulier observé dans le cas des maladies parodontales, ou parodontopathies.

Le parodonte représente l'ensemble des structures (gencive, ligament dento-alvéolaire, os alvéolaire et cément) qui assurent le soutien de la dent dans son alvéole dentaire.

Les parodontopathies se manifestent par des épisodes inflammatoires d'origine infectieuse, plus ou moins localisés, souvent récidivants, et à l'issue desquels le tissu parodontal ne se reconstruit pas correctement.

Dans cette pathologie, l'initiation de la réponse inflammatoire, et la prolifération cellulaire en réponse à la lésion infectieuse se déroulent de façon à peu près normale. En revanche, la phase de résolution est rarement satisfaisante ; on observe au mieux une réparation du tissu détruit, mais pas sa régénération complète, et chaque épisode de la maladie induit une perte tissulaire.

Les parodontopathies non traitées évoluent vers la mobilité puis la perte des dents, chez l'adulte après quarante ans. Dans la mesure où ces maladies parodontales, sous une forme plus ou moins étendue, concernent environ 10 à 15% de la population, elles ont des conséquences importantes en terme de santé publique.

Actuellement, la plupart des traitements proposés visent à débrider mécaniquement la lésion, et à réduire l'agression microbienne par l'administration d'antiseptiques, ou d'antibiotiques.

Une autre approche thérapeutique consisterait à améliorer la qualité du processus de réparation, afin de lui permettre d'aboutir à une régénération complète. Toutefois, cette approche nécessite en particulier d'être en mesure de stimuler au moment voulu la population cellulaire appropriée, pour orienter les proliférations cellulaires, et contrôler ainsi les processus de remaniement tissulaire.

Dans ce but, les Inventeurs ont étudié l'action de différents polysaccharides ; il est connu que certaines de ces molécules, comme les glycosaminoglycanes, entrent dans la composition des protéoglycanes présents à l'interface cellule/matrice extra-cellulaire, et jouent un rôle dans la régulation des fonctions cellulaires. Il est également connu que des glycosaminoglycanes sous forme soluble, par exemple l'héparine ou des dérivés du dextrane, peuvent modifier les fonctions cellulaires par l'intermédiaire de leur interaction avec différents constituants de la matrice extra-cellulaire.

Par exemple, dans le cas de l'héparine, un effet stimulateur de la prolifération cellulaire a été montré dans le cas de fibroblastes pulmonaires de hamster, de cellules épithéliales de cristallin de bovin [ULRICH et al., Biochem. Biophys. Res. Commun., 139, p. 728-732, (1986)] ou de cellules endothéliales capillaires [(SUDLALTER et al., J.Biol. Chem., 264, p. 6892-6897, (1989)].

En revanche, il a également été observé que l'héparine inhibait de façon dose-dépendante la prolifération de certains types cellulaires : cet effet anti-prolifératif a été principalement étudié dans le cas des cellules musculaires lisses (CML), pour lesquels l'inhibition apparaît pour des concentrations de 1 μg/ml d'héparine dans les milieux de culture. L'héparine s'oppose à la fois à la migration et à la prolifération des CML, mais n'affecte pas la réendothélialisation ni le volume du tissu conjonctif [CLOWES et CLOWES, Lab. Invest, 52, p. 611-615, (1985) ; CLOWES et CLOWES, Circ. Res., 58, p 839-845, (1986) ; CLOWES et al., J. Cell. Biol., 107, p. 1939-1945, (1988)].

Une inhibition de la prolifération a également été observée pour d'autres types cellulaires, comme par exemple les fibroblastes de sclérotique [DEL VECCHIO et al., Invest. Ophtalmol. Vis. Sci., 29, p. 1272-1276, (1988)], les fibroblastes 3T3 (fibroblastes d'embryons de souris conservant une inhibition de contact) [PAUL et al., Thromb. Res., 18, p. 883-888, (1980)], les cellules épithéliales du col de l'utérus de rate (LYONS-GIORADANO et al., Biochem. Biophys. Res. Commun., 148, p. 1264-1269, (1987)], les fibroblastes dermiques humains

FERRAO et MASON [Biochim. Biophys. Acta. 1180, 225-230, (1993)] ont étudié l'action de différents polysaccharides sur la prolifération de fibroblastes dermiques humains, et indiquent qu'à des concentrations de l'ordre de 100 μg/ml, l'héparine, l'héparane sulfate, le pentosane polysulfate, et un fucoïdane inhibent cette prolifération, tandis que la chondroïtine sulfate, le dermatane sulfate, et le hyaluronate n'ont pas d'effet. Il est indiqué que l'effet inhibiteur de la prolifération entraîne une stimulation de la synthèse du collagène de type I. En revanche, une inhibition de la synthèse du collagène I est observée lorsque les polysaccharides sont ajoutés à des cultures parvenues à confluence.

Il apparaît donc que l'action des polysaccharides sur les fonctions cellulaires est complexe, et peut varier selon le polysaccharide, le type cellulaire, et le tissu concernés, ainsi que selon la concentration de polysaccharide utilisée, et l'état des cellules.

Dans le cadre de recherches visant à élucider le mécanisme d'action de différents polysaccharides sur les fonctions fibroblastiques, et en particulier celles impliquées dans la régénération tissulaire, les Inventeurs se sont en particulier intéressés aux fucanes.

Les fucanes sont des polysaccharides sulfatés, entrant dans la constitution des parois cellulaires des thalles d'algues brunes (Phéophycées) ; ils sont également présents chez certains animaux marins, tels que l'oursin et le concombre de mer. Le fucane brut, également dénommé fucoïdane, obtenu par extraction acide à partir des parois cellulaires des thalles d'algues brunes, est constitué d'une population hétérogène de molécules, qui comprend principalement des polymères de L-fucose sulfaté, de masse molaire moyenne élevée (100 000 à 800 000 g/mol).

Les fucanes possèdent des activités biologiques variées : il a ainsi été montré qu'ils possédaient des activités anti chute des cheveux et cicatrisante sur la peau (EP-A- 0 730 867), anticoagulante, antithrombotique [T. NISHINO et T. NAGUMO, Carbohydr. Res. 229, p. 355-362, (1992) ; Demande EP 0403 377 ; S. COLLIEC et al. Thromb. Res. 64, p. 143-154 (1991) ; S. SOEDA et al. Thromb. Res. 72, p. 247-256 (1993) ; MAURAY et al. Thromb. Haemost. (5) 1280-1285 (1995)], antivirale [M. BABA et al. J. AIDS, 3, p.493-492, (1990)], antiangiogénique [R. HAHNENBERGER et A. M. JACKOBSON, Glycoconjugate J., 8, 350-353 (1991)] et anticomplémentaire [C. BLONDIN et al., Mol. Immunol., 31, p. 247-253, (1994)]. Il a également été observé qu'ils pouvaient agir comme modulateurs de la synthèse du collagène (D. LOGEART et al., J. Biomed. Materials Res., 30, 4, 1996, p. 501-508), de l'adhésion cellulaire [C.G. GLABE et al., J. Cell Sci, 61, p. 475-490, (1983)], du relargage de facteurs de croissance [D.A. BELFORT et al., J. Cell. Physiol. 157, p. 184-189, (1993)], de la prolifération de cellules tumorales [M. ELLOUALI et al., Anticancer Res., 13, p. 2011-2020 (1993) ; D.R.COOMBE et al., Int. J. Cancer, 39, pp. 82-90, (1987) ; D. RIOU et al., Anticancer Res., 16, 1213-1218 (1996)], et de cellules musculaires lisses vasculaires [LOGEART et al., Eur. J. Cell. Biol., 74, pp. 376-384 (1997)], et bloquer les interactions spermatozoïde/ovule chez différentes espèces [M.C. MAHONY et al., Contraception, 48, p. 277-289, (1993).

Des préparations de fucanes de masse molaire moyenne inférieure à 20 000, voire à 10 000 g/mole, ce qui facilite leur utilisation dans un cadre thérapeutique, ont été obtenues, par exemple par hydrolyse acide ménagée de fucane de masse molaire élevée (Brevet EP 0 403 377 au nom de IFREMER), ou par dépolymérisation radicalaire (Demande PCT WO/9708206 au nom de IFREMER et CNRS).

Dans l'exposé qui va suivre, le terme : « fucane » englobe aussi bien les fucanes de masse molaire élevée que les préparations de masse molaire plus faible obtenues à partir de ceux-ci.

Les Inventeurs ont constaté que les fucanes possédaient un profil d'activité sur les fonctions fibroblastiques différent de celui de l'héparine. Ils ont en particulier constaté, en testant ces deux polysaccharides dans les mêmes conditions, qu'alors que l'héparine inhibe à la fois la prolifération des fibroblastes dermiques et celle des fibroblastes gingivaux, les fucanes activent la prolifération des fibroblastes dermiques tout en inhibant celle des fibroblastes gingivaux. En outre, les Inventeurs ont aussi constaté que les fucanes modifient la morphologie des fibroblastes dermiques, qui s'arrondissent, alors que les fibroblastes gingivaux conservent au contraire un morphotype fibroblastique.

Les Inventeurs ont également constaté que les fucanes augmentaient la quantité de protéines dans la couche cellulaire, l'activité de la MMP-2 (gélatinase A), et inhibaient l'élastase leucocytaire.

Ces effets se manifestent aussi bien sur les fibroblastes dermiques que sur les fibroblastes gingivaux.

Les fucanes permettent de moduler l'expression et/ou de l'activité des métalloprotéinases fibroblastiques, et en particulier de la MMP-2, et d'inhiber l'élastase leucocytaire.

Les fucanes permettent ainsi de contrôler l'activité protéolytique dans les tissus conjonctifs, tels que les tissus dermiques et gingivaux, et en particulier de limiter l'activité élastase, qui présente un potentiel destructeur important vis-à-vis des structures macromoléculaires conjonctives, tout en favorisant au contraire l'activité de protéases qui participent à la reconstruction tissulaire, comme la MMP-2. La présente invention a donc pour objet l'utilisation d'au moins un fucane pour l'obtention d'un médicament destiné au traitement des pathologies parodontales et plus particulièrement à la régénération des tissus gingivaux.

Ledit médicament étant capable d'inhiber la prolifération des fibroblastes gingivaux et d'activer leur synthèse collagénique. Il permet ainsi le traitement des pathologies parodontales, par l'intermédiaire d'une amélioration de la phase de résolution.

En effet, du fait de la combinaison d'une régulation des activités protéolytique (en particulier activation de la MMP-2 et inhibition de l'élastase) avec une inhibition de la prolifération des fibroblastes gingivaux, une augmentation de la synthèse d'une matrice physiologique, et la conservation du morphotype fibroblastique, les fucanes permettent d'engager ces fibroblastes gingivaux dans la voie du remodelage, nécessaire à tout processus de réparation ou de régénération tissulaire.

Les médicaments obtenus conformément à l'invention peuvent être administrés par voie générale (orale ou parentérale). Ils peuvent également être administrés localement, sous formes de gels, crèmes, pommades, lotions, pastilles à sucer, bains de bouche, etc...

Ils peuvent également être administrés in situ par l'intermédiaire de substrats, de dispositifs résorbables ou non, tels que par exemple, des supports à libération différée, ou des éponges à délitement lent.

La présente invention sera mieux comprise à l'aide du complément de description qui va suivre, qui se réfère à des exemples démontrant l'activité de fucanes sur des fibroblastes dermiques et gingivaux.

## EXEMPLE 1 : ACTION DU FUCANE SUR LA PROLIFERATION DE FIBROBLASTES GINGIVAUX ET DERMIQUES

### Polysaccharides

Fucane :

Le fucane utilisé est une fraction de masse molaire moyenne 20 000±2000 obtenue à partir de l'algue brune marine *Ascophylum nodosum*, selon le procédé décrit dans le Brevet EP 0 403 377. Ce fucane a un taux élevé de fucose (44±5%), peu d'acides uroniques (7±3%), 28±3% de groupes sulfates et pas de protéines.

### Cellules

Les fibroblastes utilisés ont été obtenus à partir d'explants de tissus dermiques et gingivaux sains provenant de trois donneurs différents (âgés de 15 à 30 ans).

Mise en culture

Le protocole de mise en culture est le même pour les fibroblastes gingivaux et les fibroblastes dermiques.

A partir de l'explant, les cellules sont cultivées dans un milieu de culture DMEM (DULBECCO'S Modified Eagle Medium) contenant du D-glucose à 1 g/l et du GLUTAMAX à 0,862 g/l, un antibiotique (pénicilline-streptomycine 1000 U/ml), de la fungizone (amphotéricine B à 250 U/ml ; GIBCO BRL) et 20% de sérum de veau foetal (SVF) .

Les prélèvements sont sortis du milieu de transport. Rincés trois fois dans le milieu de culture, ils sont découpés finement en petits morceaux de 1 mm$^2$ environ. Ils sont disposés de telle sorte que la couche épithéliale soit orientée au-dessus et que la couche conjonctive soit en-dessous, au contact de la boîte de culture de 25 cm$^2$. La boîte est placée verticalement dans un incubateur à 37°C (95% air, 5% $CO_2$) pendant une demi-heure, de façon à favoriser l'adhésion.

Après addition d'une goutte de milieu sur chaque fragment tissulaire, la boîte est replacée dans l'incubateur pendant une nuit.

Le lendemain, le milieu de culture est remplacé par du même milieu frais. La boîte est laissée ainsi quatre jours dans l'incubateur.

Le milieu est ensuite changé tous les trois jours, et les explants sont retirés dès que les fibroblastes adhèrent à la paroi. Lorsque les fibroblastes ont envahi tout le fond de la boîte, la primo-culture est terminée.

Passages ultérieurs.

Le milieu de culture est éliminé, la boîte est rincée trois fois avec du DPBS (DULBECCO'S Phosphate Buffer Saline) pour enlever toute trace de SVF puis trypsinisée (trypsine diluée dans du DPBS à 0,05% et filtrée). Au bout de 5 minutes, les fibroblastes se décollent, s'arrondissent.

Les cellules sont réparties en 3 boîtes, dans du DMEM (10 à 12 ml) à 10% de SVF, et 1000 U/ml de pénicilline-streptomycine.

Le milieu est changé régulièrement jusqu'à colonisation complète de la nouvelle boîte.

**Protocole de prolifération cellulaire.**

Les cellules confluentes sont trypsinisées, suspendues dans du DMEM à raison de 7000 à 10000 cellules/ml puis réparties dans les puits de plaques à 24 puits. Les puits sont complétés par du milieu à 10% de SVF, et les plaques ensemencées sont replacées pendant deux heures dans l'incubateur pour permettre l'adhésion. Trois heures après l'ensemencement les milieux sont remplacés par un milieu DMEM/10% SVF (groupe témoin) ou bien DMEM/10%SVF avec 1, 10, ou 100 µg/ml du polysaccharide testé (fucane ou héparine). Les cellules sont comptées chaque jour jusqu'au 4$^{eme}$ jour.

Le pourcentage de prolifération est calculé par la relation :

$$\%P = \frac{(\text{prolifération nette avec produit - 1})}{\text{prolifération nette des témoins.}} \times 100.$$

Si la valeur calculée est positive, il y a prolifération cellulaire, si elle est négative, il y a inhibition de prolifération cellulaire.

Les résultats concernant le pourcentage de prolifération en présence de différentes concentrations en fucane sont illustrés par les Tableaux Ia (fibroblastes gingivaux) et Ib (fibroblastes dermiques) ci-dessous :

### TABLEAU Ia

| Concentration en fucane / Jours | 1 µg/ml | 10 µg/ml | 100 µg/ml |
|---|---|---|---|
| 1 | -6 | -6 | -6 |
| 2 | -9 | -36 | -32 |
| 3 | -11 | -41 | -44 |
| 4 | -22 | -39 | -55 |

### TABLEAU Ib

| Concentration en fucane / Jours | 1 µg/ml | 10 µg/ml | 100 µg/ml |
|---|---|---|---|
| 1 | 14 | 41 | 14 |
| 2 | 13 | 43 | 11 |
| 3 | 17 | 46 | 21 |
| 4 | 37 | 56 | 41 |

Ces résultats montrent que le fucane influence différemment la prolifération des fibroblastes gingivaux et des fibroblastes dermiques:

- Fibroblastes gingivaux (Tableau Ia): on peut observer une inhibition de la prolifération qui atteint son maximum en phase exponentielle de croissance. Cette inhibition semble être dose dépendante.
- Fibroblastes dermiques (Tableau Ib): les différentes expérimentations montrent un effet proprolifératif du fucane sur les cultures de fibroblastes dermiques. Cet effet est maximal au 4$^{eme}$ jour de culture, et 10 µg/ml est la concentration la plus efficace.

**EXEMPLE 2 : INFLUENCE DU FUCANE SUR L'ACTIVITE DE LA MMP-2 (GELATINASE A) ET SUR LA MORPHOLOGIE DES FIBROBLASTES :**

Les cellules sont ensemencées dans des plaques 24 puits, (7000 à 10000 cellules/ml) et cultivées en présence de DMEM/10%SVF. A confluence, le milieu est remplacé par du DMEM pour les témoins, ou du DMEM contenant les différentes concentrations en fucane (1, 10, 100 μg/ml) pendant 24 heures. Les milieux sont alors récupérés, pour la détection de l'activité gélatinolytique de la pro-MMP-2, et les cellules fixées et colorées (méthanol/GIEMSA) sont comptées et analysées par morphométrie sur station BIOCOM 200.

Détermination de l'activité gélatinolytique :

Les activités gélatinolytiques présentes dans les milieux de culture sont détectées par zymographie, après électrophorèse, en conditions non-réductrices, en gel de polyacrylamide-SDS + gélatine, puis élimination du SDS.

Les résultats sont quantifiés par analyse d'image semi-automatique sur station BIOCOM 200. Pour chaque bande apparaissant sur le gel, on détermine le produit de la densité de gris (D) par la surface de la bande (S). Ce produit, rapporté au nombre de cellules, permet d'apprécier et de comparer les différentes activités gélatinolytiques.

Les résultats sont illustrés par le tableau II ci-dessous :

TABLEAU II

|  | D×S pour 1000 cellules | | variations indexées | |
|---|---|---|---|---|
|  | Fibroblastes dermiques | fibroblastes gingivaux | FD | FG |
| Témoins | 16669±5563 | 16453±5066 | 100 | 100 |
| 1μg/ml | 36652±6829 | 21450±2880 | 219 | 130 |
| 10μg/ml | 25316±3030 | 25816±2304 | 151 | 157 |
| 100μg/ml | 27613±5710 | 22656±5656 | 165 | 137 |

Ces résultats montrent que le fucane augmente significativement, dès les plus faibles concentrations, la sécrétion de la pro-MMP2 chez les deux types fibroblastiques étudiés.

Morphométrie cellulaire

Quatre paramètres sont étudiés : le périmètre (exprimé en μm), la surface de la cellule (exprimée en μm$^2$), son diamètre équivalent (exprimé en μm), et son facteur de forme.

Le diamètre équivalent est le diamètre du plus petit cercle qui contient entièrement la cellule. Il définit la plus grande longueur de la cellule.

Le facteur de forme est déterminé par le rapport $4\pi S/P^2$, ou S représente la surface et P le périmètre : quand il tend vers zéro, il traduit une structure allongée ; lorsqu'il augmente, il traduit un arrondissement de la forme.

Les résultats ont illustrés par les Tableaux IIIa (fibroblastes gingivaux) et IIIb (fibroblastes dermiques) ci-dessous :

TABLEAU IIIa

|  | Périmètre | Surface | Diamètre | Facteur de forme |
|---|---|---|---|---|
| Témoins | 1050±234 | 6942±1682 | 93±11 | 0,08 |
| 1μg/ml | 1039±180 | 6154±1396 | 87±10 | 0,07 |
| 10μg/ml | 1182±209 | 5203±1389 | 82±10 | 0,04 |
| 100μg/ml | 910±111 | 4753±1036 | 77±9 | 0,07 |

## TABLEAU IIIb

|  | Périmètre | Surface | Diamètre | Facteur de forme |
|---|---|---|---|---|
| Témoins | 924±154 | 5446±1241 | 82±9 | 0,08 |
| 1µg/ml | 878±125 | 5665±807 | 84±6 | 0,11 |
| 10µg/ml | 919±136 | 6770±889 | 92±6 | 0,13 |
| 100µg/ml | 851±89 | 7556±1580 | 97±10 | 0,13 |

Ces résultats montrent que les fibroblastes gingivaux et dermiques réagissent différemment au fucane :

**Fibroblastes gingivaux :** La surface ainsi que le diamètre de ces cellules diminuent, alors que leur périmètre augmente. Ces paramètres permettent de calculer un facteur de forme tendant vers zéro, impliquant une cellule allongée de type fibroblastique.

**Fibroblastes dermiques**: Sous l'influence du fucane ces cellules voient leur surface et leur diamètre augmenter tandis que leur périmètre reste constant. Le facteur de forme augmente, ce qui implique des cellules s'arrondissant.

## EXEMPLE 3 : INFLUENCE DU FUCANE SUR L'ACTIVITE DE L'ELASTASE LEUCOCYTAIRE

L'activité de l'élastase leucocytaire en présence de fucane (1 µg/ml ou 10 µg/ml) ou d'héparine H108 (1UI/ml ou 10 UI/ml) est mesurée en utilisant comme substrat le peptide synthétique :

$$N-MeO-Succ-Ala-Ala-Pro-Val-PA,$$

selon le protocole décrit par BIZOT-FOULON et al. [International Journal of Cosmetic Science, 17, p. 255-264, (1995)].

Les résultats sont illustrés par le Tableau IV suivant :

TABLEAU IV

| FUCANE | | HEPARINE | |
|---|---|---|---|
| Concentration | % d'inhibition | Concentration | % d'inhibition |
| 1 µg/ml | 51,8 | 1 UI/ml | 16,95 |
| 10µg/ml | 54,5 | 10 UI/ml* | 9,25 |

*1 UI/ml= 5,8 µg/ml

Ces résultats montrent que dans le cas de l'héparine, l'inhibition de l'élastase leucocytaire est limitée, et que l'effet inhibiteur diminue lorsque la concentration en héparine augmente ; au contraire, dans le cas du fucane, l'inhibition est beaucoup plus importante, dès la concentration de 1 µg/ml.

## EXEMPLE 4 : INFLUENCE DU FUCANE SUR LA BIOSYNTHESE DES COLLAGENES FIBRILLAIRES

La biosynthèse des collagènes est mesurée après incorporation de proline tritiée ($^3$H-Pro).

Les cellules sont cultivées dans du DMEM/10%SVF jusqu'à confluence. Les milieux sont alors remplacés par du DMEM contenant de l'acide ascorbique (50 µg/ml) et de la $^3$H-Pro (25 µCi/ml) pour les témoins, ou ce même milieu additionné de fucane à différentes concentrations (1, 10, 100 µg/ml), ou bien d'héparine H108 à une concentration de 400 µg/ml. Après 24 heures les milieux et la couche cellulaire sont récupérés.

L'extraction spécifique de la proline et de l'hydroxyproline radiomarquées par la méthode de ROJKIND et GONZALES, [Anal. Biochem. 57:1-7 (1974)] permet la détermination du rapport synthèse collagénique totale/synthèse protéique totale.

Les résultats sont illustrés par les Tableaux Va (fibroblastes gingivaux) et Vb (fibroblastes dermiques) ci-dessous :

TABLEAU Va

|  | Synthèse collagène/ synthèse protéique | % de collagènes présents dans la couche cellulaire | % de protéines présentes dans la couche cellulaire |
|---|---|---|---|
| Témoins | 5 | 29 | 34 |
| 1µg | 5 | 26 | 38 |
| 10µg | 5 | 38 | 44 |
| 100µg | 5 | 40 | 51 |

TABLEAU Vb

|  | Synthèse collagène/ synthèse protéique | % de collagènes présents dans la couche cellulaire | % de protéines présentes dans la couche cellulaire |
|---|---|---|---|
| Témoins | 7 | 33 | 43 |
| 1µg | 5 | 38 | 55 |
| 10µg | 4.5 | 38 | 55 |
| 100µg | 5 | 32 | 63 |

Ces résultats montrent que les deux types de fibroblastes tendent, sous l'effet du fucane, à synthétiser une matrice se déposant préférentiellement dans la couche cellulaire. Ce dépôt matriciel paraît concerner l'ensemble des protéines et non seulement le collagène, ce qui exclut, pour les deux types cellulaires, tout risque fibrotique.

- **Fibroblastes gingivaux:** Le rapport synthèse collagénique globale/synthèse protéique globale ne varie pas. Le pourcentage de collagènes dans la couche cellulaire augmente parallèlement au pourcentage de protéines, de manière dose-dépendante.
- **Fibroblastes dermiques:** Le pourcentage des collagènes présents dans la couche cellulaire (intra- + péricellulaire) augmente à faible dose, et ne varie pas à 100µg/ml, alors que la quantité de protéines présentes dans ce compartiment augmente avec la concentration en fucane. Le rapport synthèse collagénique globale/synthèse protéique globale diminue.

Influence du fucane ou de l'héparine sur le dépôt de collagènes péricellulaires synthétisés par les fibroblastes gingivaux.

Les cellules sont cultivées comme décrit ci-dessus, en présence de proline tritiée ; on additionne du fucane à une concentration de 100 µg/ml, ou d'héparine H108 (masse molaire moyenne 21 000±2000, activité 173 UI/mg, commercialisée par CHOAY SANOFI) à une concentration de 400 µg/ml.
Pour estimer la quantité de collagène fibrillaire, on procède à l'extraction différentielle des collagènes intracellulaires et péricellulaires par le déoxycholate (qui extrait essentiellement le procollagène intracellulaire) et le SDS (qui solubilise le collagène péricellulaire accumulé dans la matrice extracellulaire).
Les résultats sont illustrés par le tableau VI ci-dessous :

TABLEAU VI

| | % collagène péricellulaire | Variation/témoins |
|---|---|---|
| Témoins | 19 | 0 |
| H108 | 29 | +35 |
| fucane | 44 | +56 |

Ces résultats permettent de confirmer que l'augmentation des collagènes dans la couche cellulaire est bien due à un dépôt matriciel (donc péricellulaire) et non à une rétention intracellulaire excessive. Ils montrent en outre que ce dépôt matriciel est plus important en présence de fucane qu'en présence d'héparine.

**Revendications**

1. Utilisation d'au moins un fucane pour l'obtention d'un médicament destiné au traitement des pathologies parodontales.

2. Utilisation selon la revendication 1, **caractérisée en ce que** ledit médicament est destiné à la régénération des tissus gingivaux.

3. Utilisation selon la revendication 1 ou 2, **caractérisée en ce que** ledit fucane est incorporé dans un dispositif à libération retardée.

**Claims**

1. Use of at least one fucan for the obtaining of a drug for the treatment of parodontal diseases.

2. Use according to claim 1, **characterized in that** said drug is for the gingival tissue regeneration.

3. Use according to claim 1 or 2, **characterized in that** said fucan is incorporated into a late-release device.

**Patentansprüche**

1. Verwendung eines mindestens Fucanes zur Herstellung eines Medikaments zur Heilbehandlung von perodontische Krankheitslehren.

2. Verwendung nach Anspruch 1, **dadurch gekennzeichnet, dass** das Medikament zur Zellenneubildung von Gingivalgeweben bestimmt ist.

3. Verwendung nach Anspruch 1 oder 2, **dadurch gekennzeichnet, dass** das Fucane in einer kontrollierten Freigabe Vorrichtung einverleiben ist.